# FASCICULE DE BREVET EUROPEEN

(11) **EP 2 370 488 B1**
(45) Date de publication et mention de la délivrance du brevet: **25.03.2020**
(21) Numéro de dépôt: 09736847.6
(22) Date de dépôt: 12.10.2009
(51) Int. Cl.: C08G 18/18, C08G 18/48

(54) **NOUVEAUX CATALYSEURS POUR LA REACTION ENTRE UN ISOCYANATE ET UN ALCOOL**
NEUE KATALYSATOREN FÜR DIE REAKTION ZWISCHEN EINEM ISOCYANAT UND EINEM ALKOHOL
NOVEL CATALYSTS FOR REACTION BETWEEN AN ISOCYANATE AND AN ALCOHOL

(30) Priorité: 13.10.2008 FR 0805636
(43) Date de publication de la demande: 05.10.2011
(73) Titulaire: ELKEM SILICONES France SAS, 69003 Lyon (FR)
(72) Inventeur: MALIVERNEY, Christian, F-69690 Saint Julien sur Bibost (FR); SAINT-JALMES, Laurent, F-69390 Vourles (FR)
(74) Mandataire: Mekki, Boualem
(86) Numéro de dépôt international: PCT/EP2009/007309
(87) Numéro de publication internationale: WO 2010/043353

(56) Documents cités:
- DE-A1- 3 102 343
- US-A- 3 645 924
- US-A- 4 192 925

## Description

L'invention concerne de nouveaux catalyseurs pour la réaction entre un isocyanate et un alcool, étape clé pour la préparation de polymères polyuréthanes. Elle concerne plus spécifiquement l'utilisation de nouveaux catalyseurs qui ne soient pas à base d'étain.

Les polyuréthanes ont été initialement utilisés dans la fabrication de mousses et composés plastiques. Depuis, ces polymères se sont développés dans des domaines d'application très divers tels les élastomères, les thermoplastiques, les résines thermodurcissables, les systèmes expansés, les fibres textiles et les systèmes d'enduction (bains de couchage pour le papier, revêtements du bois, peintures automobiles, adhésifs,...).

Les polyuréthanes sont des polymères qui contiennent au moins un groupement uréthane (aussi appelé carbamate). Ce groupement est issu de la réaction entre un groupement alcool et un groupement isocyanate.

D'une manière générale, la synthèse d'un polyuréthane par réaction, non catalysée, d'un isocyanate avec un alcool primaire ou secondaire est conduite entre 50 et 100°C. Ainsi d'optimiser cette réaction, de nombreux catalyseurs ont déjà été proposés, par exemple les acides et les bases de Lewis, ainsi que de nombreux sels métalliques. Des exemples de ces catalyseurs sont décrits dans les articles suivants:
- Gambiroza-Jukic, et al Kinetic analysis of bulk polymerization of diisocyanate and polyol; J. Appl. Polym. Sci., 1993, vol 47, p 513-519,
- Wong etal., Catalysis in competing isocyanate reactions, Competing phenyl isocyanate reaction catalyzed with N,N',N"-pentamethyldipropylenetriamine; J. Polym. Sci.; Part A, Polym. Chem. Ed., 1986, vol 24, p 2877-2890 et
- Okada, H., et al The kinetics of the polyurethane-forming reaction between organic diisocyanates and glycols; Makromol. Chem., 1963, vol 66, p 91-101.

Les catalyseurs métalliques les plus utilisés sont les carboxylates d'alkylétain dont le plus connu est le dilaurate de dibutylétain. Cependant, les catalyseurs à base d'alkylétain, bien que très efficaces présentent l'inconvénient d'être toxiques (CMR-2: toxiques pour la reproduction).

Aussi cherche-t-on pour de nombreuses applications à les remplacer par des composés ne présentant pas ces inconvénients. En outre, l'industrie est toujours à l'affût de composés qui soient au moins aussi actif que le dilaurate de dibutylétain, mais qui ne soient pas à base d'étain.

L'objectif essentiel de la présente invention est donc de fournir un catalyseur pour la réaction entre un isocyanate et un alcool qui soit au moins aussi actif que le dilaurate de dibutylétain, mais qui ne soit pas à base d'étain.

Un autre objectif essentiel de la présente invention est de fournir un catalyseur qui soit utilisable pour la synthèse des polyuréthanes.

Il a maintenant été trouvé, et c'est ce qui constitue l'objet de la présente invention un nouveau procédé pour préparer un composé **A** ayant au moins une fonction uréthane comprenant une étape 1) consistant à faire réagir un composé **B** ayant au moins une fonction isocyanate avec un composé **D** ayant au moins une fonction hydroxyle en présence d'un catalyseur **C** caractérisé en ce que ledit catalyseur **C** est différent du composé **D** et est un composé organique non silylé et répondant à la formule générale **(1):** 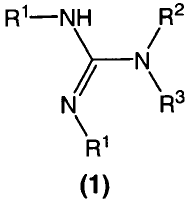 dans laquelle,
- les radicaux R¹, identiques ou différents, représentent, indépendamment l'un de l'autre, un groupe alkyle monovalent linéaire ou ramifié, un groupement cycloalkyle, un groupe (cycloalkyl)alkyle, le cycle étant substitué ou non et pouvant comprendre au moins un hétéroatome ou un groupement fluoroalkyle,
- le radical R² représente un atome d'hydrogène, un groupement alkyle monovalent linéaire ou ramifié, un groupement cycloalkyle, un groupement alkyle substitué par un cycle, substitué ou non et pouvant comprendre au moins un hétéroatome, un groupement aromatique un groupe arylalkyle, un groupement fluoroalkyle, un groupement alkylamine ou alkylguanidine, et
- le radical R³ représente un groupement alkyle monovalent linéaire ou ramifié, un groupement cycloalkyle, un groupement alkyle substitué par un cycle, substitué ou non et pouvant comprendre au moins un hétéroatome, un groupement arylalkyle, fluoroalkyle, alkylamine ou alkylguanidine,
- lorsque le radical R² n'est pas un atome d'hydrogène, les radicaux R² et R³ peuvent être liés pour former un cycle aliphatique à 3, 4, 5, 6 ou 7 chaînons éventuellement substitué par un ou plusieurs substituants, et
- avec la condition supplémentaire que les radicaux R¹, R² et R³ ne comprennent pas d'atome de silicium.

Pour atteindre cet objectif, les inventeurs ont eu le mérite de mettre en évidence, de manière tout à fait surprenante et inattendue, que l'utilisation d'un composés non silylé et répondant à la formule générale **(1)** permet de catalyser la réaction entre un isocyanate et un alcool, étape clé pour la préparation de polymères polyuréthanes.

Les composés non silylés selon l'invention et répondant à la formule générale **(1)** sont des guanidines 1,2,3-trisubstituées et 1,2,3,3-tétrasubstituées et présentent l'avantage d'être liquides, incolores et inodores.

Il est à noter qu'au moins une partie du caractère inventif de l'invention, tient à la sélection judicieuse et avantageuse des associations délimitées de composés **C** selon l'invention utilisées à titre de catalyseur.

Selon une disposition préférée de réalisation de l'invention, le catalyseur **C** est un composé organique non silylé répondant à la formule générale **(1)** décrite ci-dessus et dans laquelle:
- les radicaux R₁, identiques ou différents, et le radical R³ sont choisis, indépendamment les uns des autres, dans le groupe constitué par: un radical isopropyle, un radical cyclohexyle et un radical alkyle monovalent linéaire ou ramifié en C₁ - C₁₂.
- le radical R² représente un atome d'hydrogène, un groupe alkyle monovalent linéaire ou ramifié, un groupe cycloalkyle, un groupe alkyle substitué par un cycle, substitué ou non et pouvant comprendre au moins un hétéroatome, un groupe arylalkyle, un groupe fluoroalkyle, un groupe alkylamine ou alkylguanidine, et
- lorsque le radical R² n'est pas un atome d'hydrogène, les radicaux R² et R³ peuvent être liés pour former un cycle aliphatique à 3, 4, 5, 6 ou 7 chaînons éventuellement substitué par un ou plusieurs substituants.

Selon un autre mode de réalisation préféré le catalyseur **C** est un composé organique non silylé choisi parmi le groupe constitué par les composés **(A1)** à **(A6)** suivants:

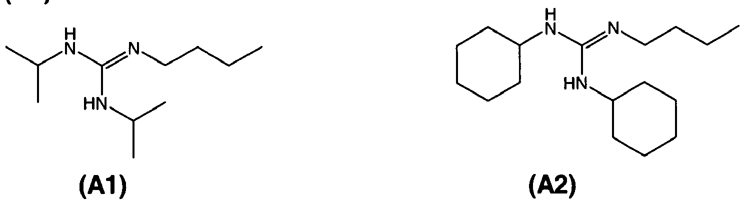

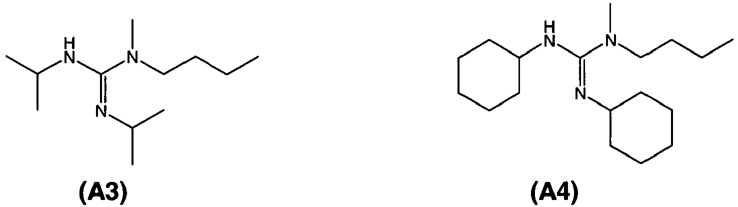

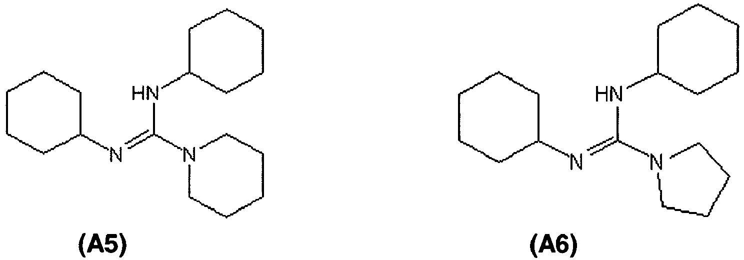

La quantité de catalyseur est avantageusement déterminée de manière à ce que la quantité est comprise entre 0,001 et 0,1% en poids par rapport à la quantité totale du mélange réactionnel et de préférence compris entre 0,005 et 0,5% en poids par rapport au poids total du mélange réactionnel.

Selon un autre mode de réalisation préféré, le procédé selon l'invention est particulièrement adapté pour la préparation de polymères polyuréthanes. Ainsi suivant une variante préférée de l'invention, le composé **A** ayant au moins une fonction uréthane est un polyuréthane, le composé **B** ayant au moins une fonction isocyanate est un diisocyanate et le composé **D** ayant au moins une fonction hydroxyle est un polyol.

Selon un autre mode de réalisation préféré, le procédé selon l'invention est caractérisé en ce que l'étape 1) consiste à faire réagir en l'absence d'humidité et en présence d'une quantité efficace du catalyseur **C** selon l'invention et tel que décrit ci-dessus, au moins un composé **B** qui est un isocyanate choisi parmi le groupe constitué par: les monoisocyanates, les diisocyanates, les polyisocyanates et leurs mélanges, et au moins un composé **D** qui est un alcool choisi parmi le groupe constitué par: les monoalcools, les diols, les polyols et leurs mélanges.

A titre d'illustration de composés **B** utile selon l'invention et ayant au moins une fonction isocyanate, on peut citer les mono, di ou polyisocyanates aromatiques, cycliques saturés ou aliphatiques bien connus de l'homme du métier, ainsi que les mélanges de ces composés.

Selon l'usage courant en chimie, lorsqu'une fonction a donné son nom a une famille de composés (en d'autres termes quand une fonction sert d'éponyme a une famille de produits, comme c'est le cas pour les isocyanates), on définit le caractère aromatique ou aliphatique selon le point d'attache de la fonction considérée.

Lorsqu'un isocyanate est situé sur un carbone de nature aliphatique, alors on considère que le compose isocyanate est lui-même de nature aliphatique. De même, lorsqu'une fonction isocyanate est rattachée au squelette par l'intermédiaire d'un carbone de nature aromatique, alors on désignera l'ensemble du monomère par l'expression isocyanate aromatique. Ainsi on considère comme:
- "aromatique", toute fonction isocyanate dont le point d'attache de l'azote est un maillon d'un cycle aromatique; et
- "aliphatique", toute fonction isocyanate dont le point d'attache de l'azote est un carbone d'hybridation sp³.

A titre d'exemples d'isocyanates aromatiques, on peut citer le diphénylméthane diisocyanate (MDI), notamment le diphénylméthane-4,4'-diisocyanate, le diphénylméthane-2,4'-diisocyanate, le toluène diisocyanate (TDI), notamment le toluène-2,4-diisocyanate et le toluène-2,6-diisocyanate.

A titre d'exemples d'isocyanates aliphatiques, on peut citer l'hexaméthylène diisocyanate (HMDI), le 1,3-tétraméthylxylylène diisocyanate, l'isophorone diisocyanate et le dicyclohexaméthylméthane diisocyanate.

A titre d'exemples de d'isocyanates cycloaliphatiques, on peut citer l'isophorone diisocyanate (IPDI).

Pour la préparation d'un polymère polyuréthane linéaire, on peut ainsi, de façon classique, faire réagir un diisocyanate et un diol. Les réactions en cause peuvent se développer selon de nombreuses variantes: il doit intervenir au moins deux réactifs de type différent (isocyanate/alcool), ces réactifs peuvent être mono ou difonctionnels.

A titre d'exemple de composé **D** ayant au moins une fonction hydroxyle, on peut citer, sans intention de s'y limiter, les polyols comme le glycérol, le polyglycérol, le glycol, le propylène glycol, les glycols comprenant de 2 à 10 atomes de carbone, de préférence de 2 à 6 atomes de carbone tels que l'éthylène glycol, le diéthylène glycol, le 1,4 butanediol, le 1,5 pentanediol, le 1,6 hexanediol, le 1,10-décanediol, le 1,3-propanediol, le dipropylène glycol polyéthylène glycol, le polypropylène glycol, le néopentylglycol, le pentaérythritol, l'hydroxypivalate de néopentylglycol, le dipentaérythritol, le triméthylol- propane, le butyl-2 éthyl-2 propanediol-1,3, le sorbitol, le mannitol, le xylitol et le mésoérythritol. On peut également utiliser des esters de ces diols ou des polyesters polyols ainsi que des polyol polyéthers.

De manière connue, les polyesters polyols sont généralement choisis parmi les polyesters polyols aliphatiques et aromatiques, et les mélanges de ces composés.

A titre d'exemple, on peut citer les polyesters polyols résultant de la condensation de polyols aliphatiques, cycliques ou aromatiques tels que le 1,2-éthanediol, le 1,2-propanediol, le 1,3-propanediol, le glycérol, le triméthylolpropane, le 1,6-hexanediol, le 1,2,6-hexanetriol, le butènediol, le sucrose, le glucose, le sorbitol, le pentaérythritol, le mannitol, la triéthanolamine, la N-méthyl diéthanolamine et les mélanges de ces composés avec un diacide carboxylique tel que l'acide 1,6-hexanedioïque, l'acide dodécanedioïque, l'acide azélaïque, l'acide sébacique, l'acide adipique, l'acide 1,18-octadécanedioïque, l'acide phtalique, l'acide succinique et les mélanges de ces diacides, un anhydride insaturé tel que l'anhydride maléique ou phtalique, ou un homopolymère de lactone telle que la ε-caprolactone.

Les polyesters polyols sont généralement obtenus par l'utilisation d'un excès d'alcool di- ou polyfonctionnel dans leur polyestérification avec des diacides ou des anhydrides carboxyliques.

Les polyols polyéthers sont en général obtenus par la polyaddition, anionique ou cationique, de monomères cycliques, comme l'oxyde d'éthylène, l'oxyde de propylène ou encore le tétrahydrofurane.

Les masses molaires des polyols polyéthers utilisés dans la synthèse de polyuréthanes varient en général de 250 à 8000. Leur fonctionnalité peut aller de 2 à 7 en fonction de la nature de la molécule utilisée comme amorceur. Les groupements terminaux de ces diols polyéthers peuvent être primaires ou secondaires.

Selon un autre mode de réalisation préféré, le procédé selon l'invention est caractérisé en ce que l'étape 1) est effectuée en présence d'un agent porogène.

De nombreux agents porogènes sont bien connus dans la technique et on les utilise à des quantités variées selon la taille cellulaire désirée dans le produit final qui est une mousse polyuréthane. Le plus économique de ces agents est l'eau mais on utilise souvent, seuls ou mélangés avec de l'eau, des alcanes à courtes chaînes halogénées portant du chlore et/ou du fluor. Les agents porogènes sont souvent utilisés à des quantités s'élevant jusqu'à 50 % du poids du polyol.

Bien entendu diverses modifications peuvent être apportées par l'homme de l'art aux procédés qui viennent d'être décrits uniquement à titre d'exemples non limitatifs sans sortir du cadre de l'invention.

D'autres avantages et caractéristiques de la présente invention apparaîtront à la lecture des exemples suivants donnés à titre illustratif nullement limitatif.

### EXEMPLES

### I) Préparation des catalyseurs selon l'invention

### a) 1-butyl-2,3-diisopropylguanidine (A1) :

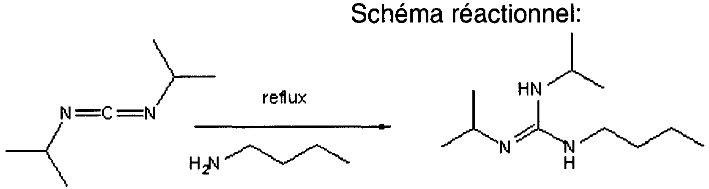

Un mélange de 33 g de N-butylamine (0,45 mol) et de 19 g de diisopropylcarbodiimide (0,15 mol) est chauffé à reflux 3h30. L'analyse par CPG montre alors une conversion supérieure à 99.5 % de la diisopropylcarbodiimide. Le mélange final incolore est concentré à 60°C sous 20 mbar pendant 2 h pour donner 29 g d'un liquide incolore et pratiquement inodore de faible viscosité, correspondant à la guanidine attendue (rendement 96.7 %).
RMN ¹H/CDCl₃ (ppm) : 0.93 (3 H, t), 1.14 (12 H, d), 1.37 (2 H, sex), 1.52 (2 H, quint), 3.01 (2 H, t), 3.57 (2 H, m).

### b) 1-butyl-2,3-diisopropyl-1-méthylguanidine (A2) :

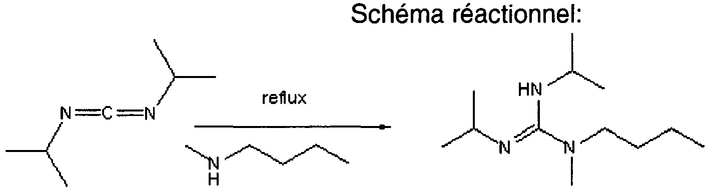

Un mélange de 32.68 g de N-butyl-N-méthylamine (0,375 mol) et de 23.66 g de diisopropylcarbodiimide (0,1875 mol) est chauffé à reflux 3h. L'analyse par CPG montre alors une conversion supérieure à 99.5 % de la diisopropylcarbodiimide. Le mélange final incolore est concentré à 60°C sous 5 mbar pendant 2 h pour donner 40 g d'un liquide incolore et pratiquement inodore de faible viscosité, correspondant à la guanidine attendue (rendement 100 %).
RMN ¹H/CDCl₃ (ppm) : 0.88 (3 H, t), 1.06 (12 H, d), 1.26 (2 H, sex), 1.46 (2 H, quint), 2.67 (3 H, s), 3.05 (2 H, t), 3.35 (2 H, m).

### c) 1-butyl-2,3-dicyclohexylguanidine (A3) RN-CAS = 60006-40-8

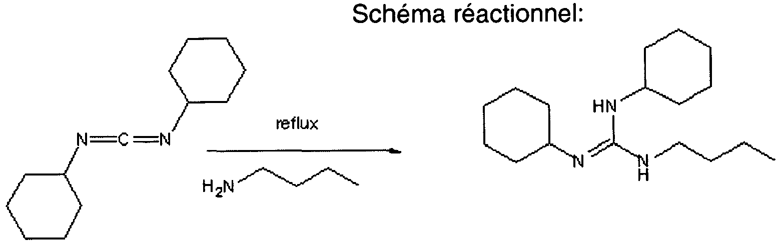

Un mélange de 15.69 g de N-butylamine (0,214 mol) et de 22.13 g de dicyclohexylcarbodiimide (0,107 mol) est chauffé à reflux 2h. L'analyse par CPG montre alors une conversion supérieure à 99.6 % de la dicyclohexylcarbodiimide. Le mélange final incolore est concentré à 60°C sous 1 mbar pendant 2 h pour donner 29.7 g d'un liquide incolore et pratiquement inodore moyennement visqueux, correspondant à la guanidine attendue (rendement 99 %).

### d) 1-butyl-2,3-dicyclohexyl-1-méthylguanidine (A4):

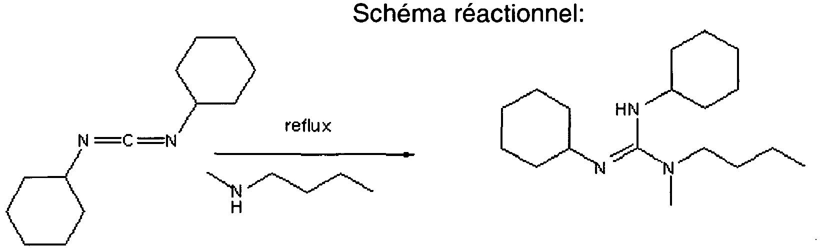

Un mélange de 17.78 g de N-butyl-N-méthylamine (0,204 mol) et de 21.05 g de dicyclohexylcarbodiimide (0,102 mol) est chauffé à reflux 3h. L'analyse par CPG montre alors une conversion supérieure à 99.5 % de la dicyclohexylcarbodiimide. Le mélange final incolore est concentré à 60°C sous 1 mbar pendant 2 h pour donner 29.9 g d'un liquide incolore et pratiquement inodore moyennement visqueux, correspondant à la guanidine attendue (rendement 99.7 %).
RMN ¹H/CDCl₃ (ppm) : 0.89 (3 H, t), 1-1.4 (10 H, m), 1.47 (2 H, quint), 1.5-2 (12 H, plusieurs m), 2.67 (3 H, s), 2.90 (1 H, m), 2.97 (1 H, m), 3.06 (2 H, t).

### e) 1,2-Dicyclohexyl-3-pipéridylguanidine (A5) RN-CAS 60006-25-9 :

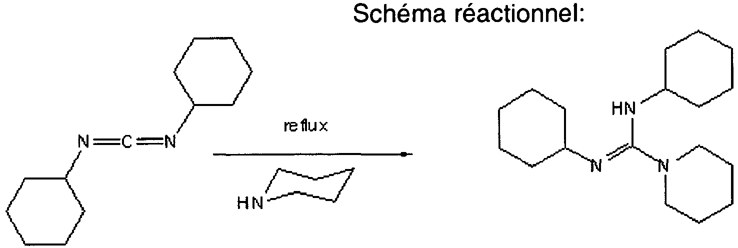

Un mélange de 11.69 g de pipéridine (0,137 mol) et de 14.16 g de dicyclohexylcarbodiimide (0,0686 mol) est chauffé à reflux 3h30. L'analyse par CPG montre alors une conversion supérieure à 99.7 % de la dicyclohexylcarbodiimide. Le mélange final incolore est concentré à 60°C sous 1 mbar pendant 2 h pour donner 19.9 g d'un liquide incolore et pratiquement inodore trés visqueux, correspondant à la guanidine attendue (rendement 99.5 %).

### f) 1,2-Dicyclohexyl-3-pyrrolidylguanidine (A6) RN-CAS 60006-28-2 :

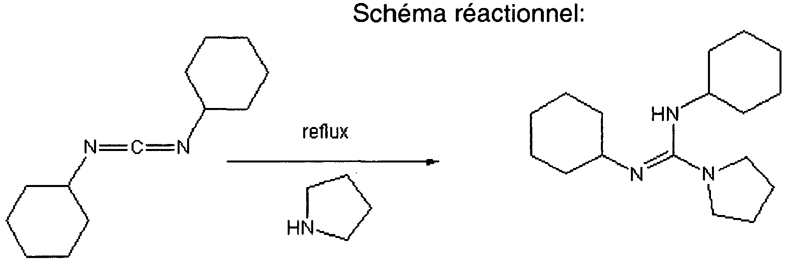

Un mélange de 19.2 g de pyrrolidine (0,27 mol) et de 18.6 g de dicyclohexylcarbodiimide (0,09 mol) est chauffé à reflux 4h. L'analyse par CPG montre alors une conversion supérieure à 99.8 % de la dicyclohexylcarbodiimide. Le mélange final incolore est concentré à 60°C sous 1 mbar pendant 1 h pour donner 24.9 g d'un liquide incolore et pratiquement inodore moyennement visqueux, correspondant à la guanidine attendue (rendement 99.6 %).

### Conditions opératoires :

On prépare une formulation composée de 2,4-TDI (2,4 diisocyanato-1-méthylbenzène) et de polypropylène glycol de masse 2000 g/mol, avec un rapport molaire OH / NCO qui est égal à 0,5. A cette formulation on ajoute le catalyseur. Les essais sont effectués en réacteur double-enveloppe, à 60°C sous ciel d'azote.

Afin de pouvoir établir des comparaisons avec le catalyseur comparatif (catalyseur à l'étain, dilaurate de dibutylétain ou DBTL), les catalyseurs **(A2)** et **(A4)** et le comparatif (DBTL) sont mise en œuvre par ajout de 0,01% en poids par rapport au poids total du mélange.

On effectue un suivi cinétique en dosant les fonctions -N=C-O (NCO) de manière habituelle, selon la norme AFNOR NF T 52-132 (Septembre 1988) parfois désigné par méthode à la dibutylamine. Le principe de ce dosage est basé sur la réaction des groupements isocyanate avec la di-n-butylamine en excès. L'amine est introduite en solution dans le toluène (1N). Le temps de réaction est de 15 minutes à température ambiante. L'excès de di-n-butylamine est ensuite dosé par titrage avec l'acide chlorhydrique (1N). Le vert de bromocrésol est utilisé comme indicateur.

On observe que:
a) un taux de 20% de conversion des fonctions isocyanates est atteint après:
   - quelques minutes pour le DBTL (comparatif), et
   - 50 mn et 55 mn pour les catalyseurs **(A2)** et **(A4)** respectivement;
b) un taux de 50% de conversion des fonctions isocyanates est atteint après:
   - 10 mn pour le DBTL (comparatif), et
   - 180 mn et 205 mn pour les catalyseurs **(A2)** et **(A4)** respectivement;

A noter que le retard de la réaction avec les catalyseurs **(A2)** et **(A4)** par rapport au comparatif présente un intérêt car dans certaines applications un temps de latence de la réticulation est souhaité.

La distribution de masses moléculaires obtenue et l'utilisation d'une double détection en chromatographie par perméation de gel ou "CPG" (réfractomètre (RI) et UV) permet d'affirmer l'efficacité des catalyseurs **(A2)** et **(A4)** selon l'invention.

Ce résultat est satisfaisant et montre la viabilité d'un catalyseur sans étain selon l'invention. De plus, comme la cinétique de formation du polymère polyuréthane étant légèrement plus lente avec le catalyseur selon l'invention, cela présente l'avantage de donner plus de temps pour une étape de mise en forme du polymère polyuréthane.

## Revendications

1. Procédé pour préparer un composé **A** ayant au moins une fonction uréthane comprenant une étape 1) consistant à faire réagir un composé **B** ayant au moins une fonction isocyanate avec un composé **D** ayant au moins une fonction hydroxyle en présence d'un catalyseur **C caractérisé en ce que** ledit catalyseur **C** est différent du composé **D** et est un composé organique non silylé et répondant à la formule générale **(1):** 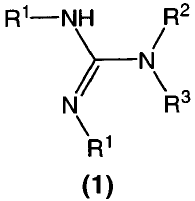 dans laquelle,
- les radicaux R¹, identiques ou différents, représentent, indépendamment l'un de l'autre, un groupe alkyle monovalent linéaire ou ramifié, un groupement cycloalkyle, un groupe (cycloalkyl)alkyle, le cycle étant substitué ou non et pouvant comprendre au moins un hétéroatome ou un groupement fluoroalkyle,
- le radical R² représente un atome d'hydrogène, un groupement alkyle monovalent linéaire ou ramifié, un groupement cycloalkyle, un groupement alkyle substitué par un cycle, substitué ou non et pouvant comprendre au moins un hétéroatome, un groupement aromatique un groupe arylalkyle, un groupement fluoroalkyle, un groupement alkylamine ou alkylguanidine, et
- le radical R³ représente un groupement alkyle monovalent linéaire ou ramifié, un groupement cycloalkyle, un groupement alkyle substitué par un cycle, substitué ou non et pouvant comprendre au moins un hétéroatome, un groupement arylalkyle, fluoroalkyle, alkylamine ou alkylguanidine,
- lorsque le radical R² n'est pas un atome d'hydrogène, les radicaux R² et R³ peuvent être liés pour former un cycle aliphatique à 3, 4, 5, 6 ou 7 chaînons éventuellement substitué par un ou plusieurs substituants, et
- avec la condition supplémentaire que les radicaux R¹, R² et R³ ne comprennent pas d'atome de silicium.

2. Procédé selon la revendication 1, **caractérisé en ce que** le catalyseur **C** est un composé organique non silylé répondant à la formule générale **(1)** et dans laquelle:
- les radicaux R₁, identiques ou différents, et le radical R₃ sont choisis, indépendamment les uns des autres, dans le groupe constitué par: un radical isopropyle, un radical cyclohexyle et un radical alkyle monovalent linéaire ou ramifié en C₁ - C₁₂.
- le radical R² représente un atome d'hydrogène, un groupe alkyle monovalent linéaire ou ramifié, un groupe cycloalkyle, un groupe alkyle substitué par un cycle, substitué ou non et pouvant comprendre au moins un hétéroatome, un groupe arylalkyle, un groupe fluoroalkyle, un groupe alkylamine ou alkylguanidine, et
- lorsque le radical R² n'est pas un atome d'hydrogène, les radicaux R² et R³ peuvent être liés pour former un cycle aliphatique à 3, 4, 5, 6 ou 7 chaînons éventuellement substitué par un ou plusieurs substituants.

3. Procédé selon la revendication 1 dans lequel le catalyseur **C** est un composé organique non silylé choisi parmi le groupe constitué par les composés **(A1)** à **(A6)** suivants:

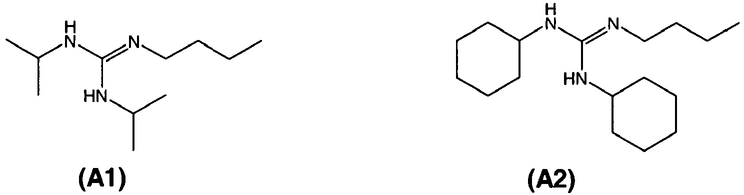

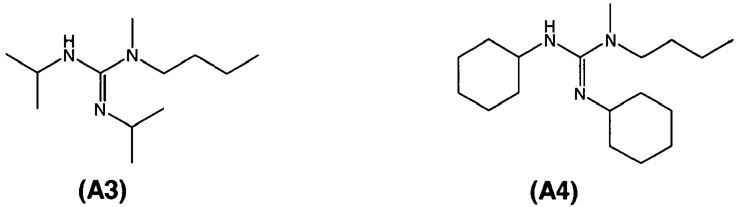

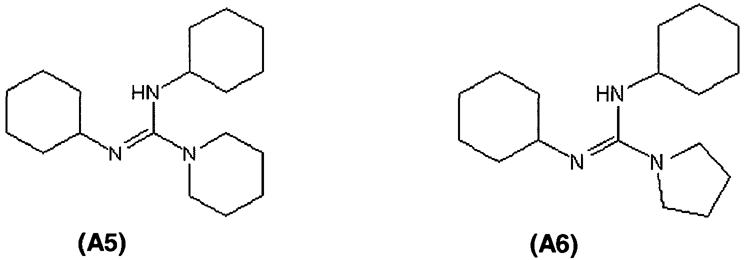

4. Procédé selon l'une des revendications précédentes **caractérisé en ce que** le composé **A** ayant au moins une fonction uréthane est un polyuréthane, le composé **B** ayant au moins une fonction isocyanate est un diisocyanate et le composé **D** ayant au moins une fonction hydroxyle est un polyol.

5. Procédé selon l'une quelconque des revendications 1 à 3 dans lequel l'étape 1) consiste à faire réagir en l'absence d'humidité et en présence d'une quantité efficace du catalyseur **C** selon l'invention et tel que décrit ci-dessus, au moins un composé **B** qui est un isocyanate choisi parmi le groupe constitué par: les monoisocyanates, les diisocyanates et leurs mélanges, et au moins un composé **D** qui est un alcool choisi parmi le groupe constitué par: les monoalcools, les diols, les polyols et leurs mélanges.

6. Procédé selon l'une des revendications précédentes **caractérisé en ce que** le composé **B** est un diisocyanate choisi parmi le groupe constitué par les composés suivants: le diphénylméthane diisocyanate (MDI), notamment le diphénylméthane-4,4'-diisocyanate, le diphénylméthane-2,4'-diisocyanate, le toluène diisocyanate (TDI), notamment le toluène-2,4-diisocyanate, le toluène-2,6-diisocyanate, l'hexaméthylène diisocyanate (HMDI), le 1,3-tétraméthylxylylène diisocyanate, l'isophorone diisocyanate, dicyclohexaméthylméthane diisocyanate et l'isophorone diisocyanate (IPDI).

7. Procédé selon la revendication 4 **caractérisé en ce que** le composé **D** est un polyester polyol.

8. Procédé selon l'une des revendications précédentes **caractérisé en ce que** l'étape 1) est effectuée en présence d'un agent porogène, de préférence ledit agent porogène est l'eau

## Patentansprüche

1. Verfahren zur Herstellung einer Verbindung **A** mit mindestens einer Urethanfunktion, umfassend einen Schritt 1), der darin besteht, dass man eine Verbindung **B** mit mindestens einer Isocyanatfunktion mit einer Verbindung **D** mit mindestens einer Hydroxylfunktion in Gegenwart eines Katalysators **C** umsetzt, **dadurch gekennzeichnet, dass** der Katalysator **C** von der Verbindung **D** verschieden ist und eine nichtsilylierte organische Verbindung ist und der allgemeinen Formel **(1)** entspricht: 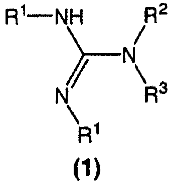 worin
- die Reste R¹, die gleich oder verschieden sind, unabhängig voneinander für eine lineare oder verzweigte einwertige Alkylgruppe, eine Cycloalkylgruppe, eine (Cycloalkyl)alkylgruppe, wobei der Ring substituiert oder unsubstituiert ist und mindestens ein Heteroatom umfassen kann, oder eine Fluoralkylgruppe stehen,
- der Rest R² für ein Wasserstoffatom, eine lineare oder verzweigte einwertige Alkylgruppe, eine Cycloalkylgruppe, eine durch einen Ring, der substituiert oder unsubstituiert ist und mindestens ein Heteroatom umfassen kann, substituierte Alkylgruppe, eine aromatische Gruppe, eine Arylalkylgruppe, eine Fluoralkylgruppe, eine Alkylamingruppe oder eine Alkylguanidingruppe steht und
- der Rest R³ für eine lineare oder verzweigte einwertige Alkylgruppe, eine Cycloalkylgruppe, eine durch einen Ring, der substituiert oder unsubstituiert ist und mindestens ein Heteroatom umfassen kann, substituierte Alkylgruppe, eine Arylalkylgruppe, eine Fluoralkylgruppe, eine Alkylamingruppe oder eine Alkylguanidingruppe steht,
- dann, wenn der Rest R² nicht für ein Wasserstoffatom steht, die Reste R² und R³ unter Bildung eines 3-, 4-, 5-, 6- oder 7-gliedrigen aliphatischen Rings, der gegebenenfalls durch einen oder mehrere Substituenten substituiert ist, verbunden sein können, und
- mit der zusätzlichen Maßgabe, dass die Reste R¹, R² und R³ kein Siliciumatom umfassen.

2. Verfahren nach Anspruch 1, **dadurch gekennzeichnet, dass** es sich bei dem Katalysator **C** um eine nichtsilylierte organische Verbindung handelt, die der allgemeinen Formel **(1)** entspricht und worin:
- die Reste R¹, die gleich oder verschieden sind, und der Rest R³ unabhängig voneinander aus der Gruppe bestehend aus einem Isopropylrest, einem Cyclohexylrest und einem linearen oder verzweigten einwertigen C₁-C₁₂-Alkylrest ausgewählt sind,
- der Rest R² für ein Wasserstoffatom, eine lineare oder verzweigte einwertige Alkylgruppe, eine Cycloalkylgruppe, eine durch einen Ring, der substituiert oder unsubstituiert ist und mindestens ein Heteroatom umfassen kann, substituierte Alkylgruppe, eine Arylalkylgruppe, eine Fluoralkylgruppe, eine Alkylamingruppe oder eine Alkylguanidingruppe steht und
- dann, wenn der Rest R² nicht für ein Wasserstoffatom steht, die Reste R² und R³ unter Bildung eines 3-, 4-, 5-, 6- oder 7-gliedrigen aliphatischen Rings, der gegebenenfalls durch einen oder mehrere Substituenten substituiert ist, verbunden sein können.

3. Verfahren nach Anspruch 1, wobei es sich bei dem Katalysator **C** um eine nichtsilylierte organische Verbindung handelt, die aus der Gruppe bestehend aus den folgenden Verbindungen **(A1)** bis **(A6)** ausgewählt ist:

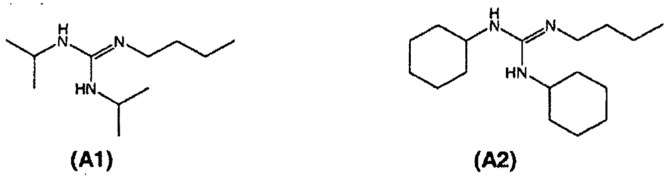

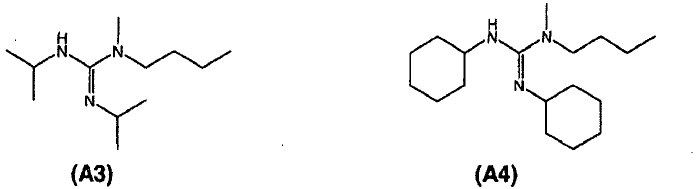

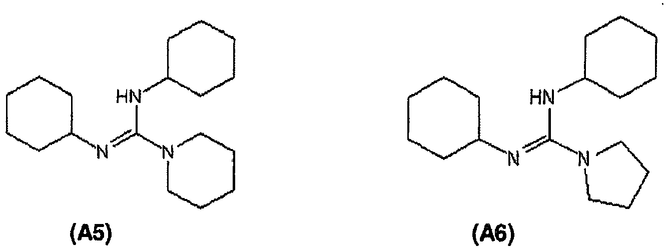

4. Verfahren nach einem der vorhergehenden Ansprüche, **dadurch gekennzeichnet, dass** es sich bei der Verbindung **A** mit mindestens einer Urethanfunktion um ein Polyurethan handelt, es sich bei der Verbindung **B** mit mindestens einer Isocyanatfunktion um ein Diisocyanat handelt und es sich bei der Verbindung **D** mit mindestens einer Hydroxylfunktion um ein Polyol handelt.

5. Verfahren nach einem der Ansprüche 1 bis 3, wobei der Schritt 1) darin besteht, dass man in Abwesenheit von Feuchtigkeit und in Gegenwart einer wirksamen Menge des erfindungsgemäßen Katalysators **C** gemäß obiger Beschreibung mindestens eine Verbindung **B,** bei der es sich um ein Isocyanat aus der Gruppe bestehend aus Monoisocyanaten, Diisocyanaten und Mischungen davon handelt, und mindestens eine Verbindung **D,** bei der es sich um einen Alkohol aus der Gruppe bestehend aus Monoalkoholen, Diolen, Polyolen und Mischungen davon handelt, umsetzt.

6. Verfahren nach einem der vorhergehenden Ansprüche, **dadurch gekennzeichnet, dass** es sich bei der Verbindung **B** um ein Diisocyanat aus der Gruppe bestehend aus den folgenden Verbindungen handelt: Diphenylmethandiisocyanat (MDI), insbesondere 4,4'-Diphenylmethandiisocyanat, 2,4'-Diphenylmethandiisocyanat, Toluoldiisocyanat (TDI), insbesondere 2,4-Toluoldiisocyanat, 2,6-Toluoldiisocyanat, Hexamethylendiisocyanat (HMDI), 1,3-Tetramethylxylylendiisocyanat, Isophorondiisocyanat, Dicyclohexamethylmethandiisocyanat und Isophorondiisocyanat (IPDI).

7. Verfahren nach Anspruch 4, **dadurch gekennzeichnet, dass** es sich bei der Verbindung **D** um ein Polyesterpolyol handelt.

8. Verfahren nach einem der vorhergehenden Ansprüche, **dadurch gekennzeichnet, dass** Schritt 1) in Gegenwart eines Porenbildners durchgeführt wird, wobei es sich bei dem Porenbildner vorzugsweise um Wasser handelt.

## Claims

1. Process for preparing a compound **A** having at least one urethane functional group, comprising a stage 1) which consists in reacting a compound **B,** having at least one isocyanate functional group, with a compound **D,** having at least one hydroxyl functional group, in the presence of a catalyst **C, characterized in that** said catalyst **C** is different from the compound **D** and is a non-silyl organic compound corresponding to the general formula **(1):** 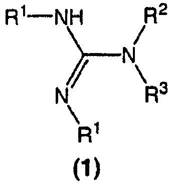 in which:
- the R¹ radicals, which are identical or different, represent, independently of one another, a linear or branched monovalent alkyl group, a cycloalkyl group, a (cycloalkyl)alkyl group, the ring being substituted or unsubstituted and being able to comprise at least one heteroatom, or a fluoroalkyl group,
- the R² radical represents a hydrogen atom, a linear or branched monovalent alkyl group, a cycloalkyl group, an alkyl group substituted by a ring, which is substituted or unsubstituted and which can comprise at least one heteroatom, an aromatic group, an arylalkyl group, a fluoroalkyl group or an alkylamine or alkylguanidine group, and
- the R³ radical represents a linear or branched monovalent alkyl group, a cylcoalkyl group, an alkyl group substituted by a ring, which is substituted or unsubstituted and which can comprise at least one heteroatom, or an arylalkyl, fluoroalkyl, alkylamine or alkylguanidine group,
- when the R² radical is not a hydrogen atom, the R² and R³ radicals can be linked up to form a 3-, 4-, 5-, 6- or 7-membered aliphatic ring optionally substituted by one or more substituents, and
- with the additional condition that the R¹, R² and R³ radicals do not comprise a silicon atom.

2. Process according to Claim 1, **characterized in that** the catalyst **C** is a non-silyl organic compound corresponding to the general formula **(1)** and in which:
- the R¹ radicals, which are identical or different, and the R³ radical are chosen, independently of one another, from the group consisting of: an isopropyl radical, a cyclohexyl radical and a linear or branched monovalent C₁-C₁₂ alkyl radical,
- the R² radical represents a hydrogen atom, a linear or branched monovalent alkyl group, a cycloalkyl group, an alkyl group substituted by a ring, which is substituted or unsubstituted and which can comprise at least one heteroatom, an arylalkyl group, a fluoroalkyl group or an alkylamine or alkylguanidine group, and
- when the R² radical is not a hydrogen atom, the R² and R³ radicals can be linked up to form a 3-, 4-, 5-, 6- or 7-membered aliphatic ring optionally substituted by one or more substituents.

3. Process according to Claim 1, in which the catalyst **C** is a non-silyl organic compound chosen from the group consisting of the following compounds **(A1)** to **(A6):**

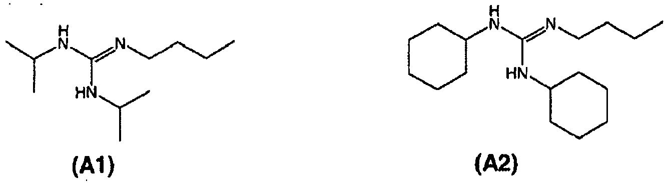

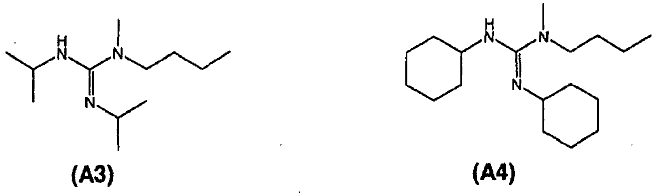

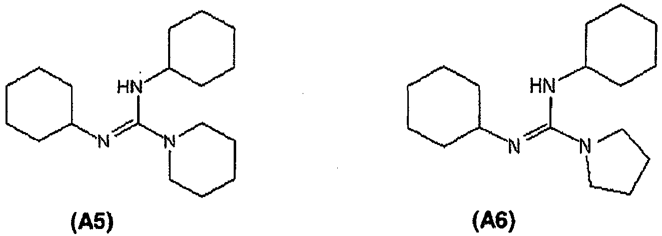

4. Process according to any one of the preceding claims, **characterized in that** the compound **A** having at least one urethane functional group is a polyurethane, the compound **B** having at least one isocyanate functional group is a diisocyanate and the compound **D** having at least one hydroxyl functional group is a polyol.

5. Process according to any one of Claims 1 to 3, in which stage **1**) consists in reacting, in the absence of moisture and in the presence of an effective amount of the catalyst **C** according to the invention and as described above, at least one compound **B,** which is an isocyanate chosen from the group consisting of: monoisocyanates, diisocyanates and their mixtures, and at least one compound **D,** which is an alcohol chosen from the group consisting of: monoalcohols, diols, polyols and their mixtures.

6. Process according to one of the preceding claims, **characterized in that** the compound **B** is a diisocyanate chosen from the group consisting of the following compounds: diphenylmethane diisocyanate (MDI), in particular 4,4'-diphenylmethane diisocyanate or 2,4'-diphenylmethane diisocyanate, toluene diisocyanate (TDI), in particular 2,4-toluene diisocyanate or 2,6-toluene diisocyanate, hexamethylene diisocyanate (HMDI), 1,3-tetramethylxylylene diisocyanate, isophorone diisocyanate, dicyclohexamethylmethane diisocyanate and isophorone diisocyanate (IPDI).

7. Process according to Claim 4, **characterized in that** the compound **D** is a polyester polyol.

8. Process according to one of the preceding claims, **characterized in that** stage 1) is carried out in the presence of an expanding agent, said expanding agent preferably being water.
